# EUROPEAN PATENT APPLICATION

(11) **EP 2 426 112 A1**
(43) Date of publication of application: **07.03.2012**
(21) Application number: 10382226.8
(22) Date of filing: 09.08.2010
(51) Int. Cl.: C07D 231/22, A61P 25/16, A61P 25/00, A61K 31/4152

(54) **4-[-2-[[5-methyl-1-(2-naphtalenyl)-1h-pyrazol-3-yl]oxy]ethyl]morpholine hydrochloride polymorphs and solvates**

(71) Applicant: Laboratorios del. Dr. Esteve, S.A., 08041 Barcelona (ES)
(72) Inventor: The designation of the inventor has not yet been filed
(74) Representative: ABG Patentes, S.L.

(57) **Abstract**

The present invention relates to polymorphich forms and solvates of 4-[2-[[5-methyl-1-(2-naphthalenyl)-1 H-pyrazol-3-yl]oxy]ethyl]morpholine hydrochloride (P027), and processes for their preparation.

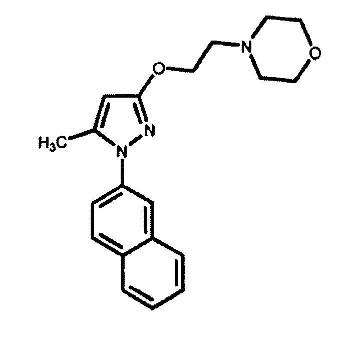

## Description

### FIELD OF THE INVENTION

The present invention relates to novel polymorphs and solvates of the hydrochloride salt of 4-[2-[[5-methyl-1-(2-naphthalenyl)-1H-pyrazol-3-yl]oxy]ethyl]morpholine (P027), processes for their preparation, their use for preparing the phase I form, and to pharmaceutical compositions comprising them.

### BACKGROUND

The search for new therapeutic agents has been greatly aided in recent years by better understanding of the structure of proteins and other biomolecules associated with target diseases. One important class of these proteins is the sigma (σ) receptor, a cell surface receptor of the central nervous system (CNS) which may be related to the dysphoric, hallucinogenic and cardiac stimulant effects of opioids. From studies of the biology and function of sigma receptors, evidence has been presented that sigma receptor ligands may be useful in the treatment of psychosis and movement disorders such as dystonia and tardive dyskinesia, and motor disturbances associated with Huntington's chorea or Tourette's syndrome and in Parkinson's disease (Walker, J.M. et al, Pharmacological Reviews, 1990, 42, 355). It has been reported that the known sigma receptor ligand rimcazole clinically shows effects in the treatment of psychosis (Snyder, S.H., Largent, B.L. J. Neuropsychiatry 1989, 1, 7). The sigma binding sites have preferential affinity for the dextrorotatory isomers of certain opiate benzomorphans, such as (+)SKF 10047, (+)cyclazocine, and (+)pentazocine and also for some narcoleptics such as haloperidol.

The sigma receptor has at least two subtypes, which may be discriminated by stereoselective isomers of these pharmacoactive drugs. SKF 10047 has nanomolar affinity for the sigma 1 (σ-1) site, and has micromolar affinity for the sigma 2 (σ-2) site. Haloperidol has similar affinities for both subtypes. Endogenous sigma ligands are not known, although progesterone has been suggested to be one of them. Possible sigma-site-mediated drug effects include modulation of glutamate receptor function, neurotransmitter response, neuroprotection, behavior, and cognition (Quirion, R. et al. Trends Pharmacol. Sci., 1992, 13:85-86). Most studies have implied that sigma binding sites (receptors) are plasmalemmal elements of the signal transduction cascade. Drugs reported to be selective sigma ligands have been evaluated as antipsychotics (Hanner, M. et al. Proc. Natl. Acad. Sci., 1996, 93:8072-8077). The existence of sigma receptors in the CNS, immune and endocrine systems have suggested a likelihood that it may serve as link between the three systems.

In view of the potential therapeutic applications of agonists or antagonists of the sigma receptor, a great effort has been directed to find selective ligands. Thus, the prior art discloses different sigma receptor ligands. 4-[2-[[5-methyl-1-(2-naphthalenyl)-1H-pyrazol-3-yl]oxy]ethyl]morpholine is one of such promising sigma receptor ligands. The compound and its synthesis are disclosed and claimed in WO2006/021462.

4-[2-[[5-methyl-1-(2-naphthalenyl)-1H-pyrazol-3-yl]oxy]ethyl]morpholine is a highly selective sigma-1 (σ-1) receptor antagonist. It has displayed strong analgesic activity in the treatment and prevention of chronic and acute pain, and particularly, neuropathic pain. The compound has a molecular weight 373.42 uma. The structural formula of the compound is:

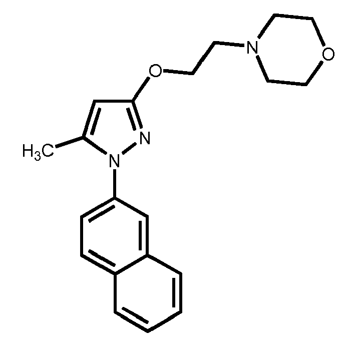

The solid state physical properties of a pharmaceutical compound can be influenced by the conditions under which the compound is obtained in solid form. Solid state physical properties include, for example, the flowability of the milled solid which affects the ease with which the compound is handled during processing into a pharmaceutical product. Another important solid state property of a pharmaceutical compound is its rate of dissolution in aqueous fluid. The rate of dissolution of an active ingredient in a patient's stomach fluid can have therapeutic consequences because it imposes an upper limit on the rate at which an orally administered active ingredient can reach the blood. The solid-state form of a compound may also affect its solubility, bioavailability, behavior on compaction, stability, or its electrostatic nature.

Polymorphism is the property of some molecules and molecular complexes to assume more than one crystalline or amorphous form in the solid state. In general, polymorphism is caused by the ability of the molecule of a substance to change its conformation or to form different inter molecular and intramolecular interactions, particularly hydrogen bonds, which is reflected in different atom arrangements in the crystal lattices of different polymorphs. Accordingly, polymorphs are distinct solids sharing the same molecular Formula, having distinct advantageous and/or disadvantageous physical properties compared to other forms in the polymorph family.

The discovery of new crystalline polymorphic or amorphous forms of a pharmaceutical compound provides an opportunity to improve the physical or performance characteristics of a pharmaceutical product in that it enlarges the repertoire of materials that a formulation scientist has available for designing, for example, a pharmaceutical dosage form of a drug with a targeted release profile or other desired characteristics.

The polymorph phase I of the the hydrochloride salt of 4-[2-[[5-methyl-1-(2-naphthalenyl)-1H-pyrazol-3-yl]oxy]ethyl]morpholine (P027) and its synthesis are disclosed and claimed in the co-pending application EP 10382025.4 filed on February 4th, 2010. Said polymorph phase I has found to be highly stable over the time with good flow and dissolution characteristics, thus providing advantageous production, handling, storage and therapeutic properties.

Other solid forms of the P027 compound are described in the co-pending application EP__________ filed at the same time that the present one.

However, there is still a need in the art for additional forms of 4-[2-[[5-methyl-1-(2-naphthalenyl)-1H-pyrazol-3-yl]oxy]ethyl]morpholine to carry out its pharmaceutical development and release its potential, and facilitate the preparation of better formulations of this active pharmaceutical ingredient. In this regard, alternative morphological forms of the compound may have widely different properties and could either become intermediates for other forms (e.g. the highly stable form I above-mentioned) or provide in themselves a still better formulation of this active pharmaceutical ingredient. Thus it is important to find such forms, having desirable properties for pharmaceutical use.

### BRIEF DESCRIPTION OF THE INVENTION

The inventors of the present invention have surprisingly found and demonstrated that new solid forms of the hydrochloride salt of 4-[2-[[5-methyl-1-(2-naphthalenyl)-1H-pyrazol-3-yl]oxy]ethyl]morpholine (P027) may achieve one or more of the above mentioned objectives. The novel polymorphic and solvated forms of P027 herein disclosed are stable over the time, have good flow and dissolution characteristics and notably may be useful as intermediates for other useful forms such as the crystalline form I of P027.

Thus, the present invention relates to polymorphic forms and solvates of P027, to their use and to several processes for their preparation.

In one embodiment, the present invention is directed to a polymorphic form of the hydrochloride salt of 4-[2-[[5-methyl-1-(2-naphthalenyl)-1H-pyrazol-3-yl]oxy]ethyl]morpholine.

Preferably, said polymorphic form is selected from the group consisting of:
- P027 phase II form, which can be characterized because it has a X-ray powder diffraction pattern showing characteristic peaks at a reflection angle [2θ] of about the values indicated below in table 1:

**Table 1**

| List of selected peaks obtained by powder X-Ray diffraction of phase II | | |
|---|---|---|
| **Angle (2θ)** | **d value (Å)** | **Intensify (%)** |
| 5,776 | 15,28888 | 30,2 |
| 11,629 | 7,60368 | 8,3 |
| 14,558 | 6,07960 | 4,4 |
| 15,737 | 5,62658 | 73,1 |
| 15,891 | 5,57256 | 37,3 |
| 16,420 | 5,39408 | 14,1 |
| 16,740 | 5,29166 | 12,7 |
| 17,441 | 5,08075 | 23,7 |
| 17,635 | 5,02527 | 100,0 |
| 18,056 | 4,90895 | 44,8 |
| 18,219 | 4,86548 | 23,3 |
| 19,232 | 4,61143 | 22,9 |
| 19,712 | 4,50004 | 4,4 |
| 20,140 | 4,40556 | 2,8 |
| 20,685 | 4,29064 | 4,3 |
| 21,135 | 4,20016 | 7,6 |
| 21,889 | 4,05717 | 33,0 |
| 22,108 | 4,01757 | 46,8 |
| 22,478 | 3,95233 | 8,8 |
| 22,763 | 3,90336 | 14,1 |
| 23,219 | 3,82779 | 13,5 |
| 23,454 | 3,78998 | 21,3 |
| 23,782 | 3,73840 | 12,6 |
| 24,689 | 3,60310 | 15,6 |
| 25,065 | 3,54983 | 10,6 |
| 25,671 | 3,46750 | 13,7 |

- P027 phase III form, which can be characterized because it has a X-ray powder diffraction pattern showing characteristic peaks at a reflection angle [2θ] of about the values indicated below in table 2:

**Table 2**

| List of selected peaks obtained by powder X-Ray diffraction of phase III | | |
|---|---|---|
| **Angle (2θ)** | **d value (Å)** | **Intensity (%)** |
| 5,437 | 16,24165 | 14,9 |
| 5,714 | 15,45508 | 31,2 |
| 10,918 | 8,09724 | 1,9 |
| 11,546 | 7,65777 | 3,4 |
| 12,704 | 6,96243 | 2,3 |
| 13,344 | 6,63006 | 7,3 |
| 13,984 | 6,32777 | 6,0 |
| 14,505 | 6,10193 | 3,7 |
| 15,606 | 5,67363 | 24,1 |
| 15,824 | 5,59613 | 61,7 |
| 16,164 | 5,47909 | 40,3 |
| 16,646 | 5,32137 | 5,1 |
| 17,333 | 5,11195 | 8,3 |
| 17,837 | 4,96880 | 80,1 |
| 18,719 | 4,73663 | 62,4 |
| 18,878 | 4,69703 | 38,3 |
| 19,236 | 4,61037 | 10,2 |
| 19,533 | 4,54088 | 25,1 |
| 20,142 | 4,40496 | 20,3 |
| 20,689 | 4,28973 | 15,8 |
| 21,337 | 4,16103 | 4,2 |
| 22,008 | 4,03562 | 13,9 |
| 22,929 | 3,87545 | 19,6 |
| 23,596 | 3,76747 | 100,0 |
| 24,748 | 3,59457 | 10,1 |
| 25,064 | 3,55008 | 35,7 |
| 25,207 | 3,53024 | 47,7 |
| 25,737 | 3,45874 | 25,5 |
| 26,148 | 3,40521 | 66,9 |

- P027 phase IV form, which can be characterized because it has a X-ray powder diffraction pattern showing characteristic peaks at a reflection angle [2θ] of about the values indicated below in table 3:

**Table 3**

| List of selected peaks obtained by powder X-Ray diffraction of phase IV | | |
|---|---|---|
| **Angle (2θ)** | **d value (Å)** | **Intensity (%)** |
| 5,805 | 15,21150 | 51,5 |
| 11,685 | 7,56709 | 30,4 |
| 15,559 | 5,69074 | 84,9 |
| 15,804 | 5,60321 | 7,1 |
| 16,397 | 5,40173 | 49,5 |
| 16,879 | 5,24838 | 47,7 |
| 17,357 | 5,10514 | 39,2 |
| 17,465 | 5,07372 | 42,4 |
| 17,621 | 5,02921 | 66,8 |
| 19,112 | 4,64012 | 100,0 |
| 19,435 | 4,56373 | 3,8 |
| 19,923 | 4,45292 | 16,1 |
| 21,224 | 4,18278 | 10,9 |
| 21,987 | 4,03934 | 83,5 |
| 22,167 | 4,00707 | 45,4 |
| 22,412 | 3,96379 | 33,8 |
| 22,852 | 3,88840 | 18,0 |
| 23,059 | 3,85401 | 14,4 |
| 23,359 | 3,80517 | 61,8 |
| 23,855 | 3,72720 | 13,6 |
| 24,092 | 3,69105 | 29,7 |
| 25,722 | 3,46066 | 16,4 |
| 26,054 | 3,41730 | 10,8 |
| 26,649 | 3,34237 | 16,3 |
| 27,780 | 3,20885 | 4,2 |

In another embodiment, the present invention is directed to a solvate of the hydrochloride salt of 4-[2-[[5-methyl-1-(2-naphthalenyl)-1H-pyrazol-3-yl]oxy]ethyl]morpholine. Preferably, said solvate is selected from the group consisting of:
- P027 dioxane solvate, which can be characterized because it has a X-ray powder diffraction pattern showing characteristic peaks at a reflection angle [2θ] of about the values indicated below in table 4:

**Table 4**

| List of selected peaks obtained by powder X-Ray diffraction of the dioxane solvate | | |
|---|---|---|
| **Angle (2θ)** | **d value (Å)** | **Intensity (%)** |
| 4,734 | 18,65133 | 12,1 |
| 9,317 | 9,48417 | 17,2 |
| 11,390 | 7,76280 | 14,5 |
| 13,614 | 6,49913 | 6,8 |
| 14,290 | 6,19322 | 6,6 |
| 14,815 | 5,97468 | 47,7 |
| 16,211 | 5,46334 | 17,2 |
| 16,432 | 5,39027 | 15,0 |
| 16,782 | 5,27852 | 5,4 |
| 17,741 | 4,99534 | 4,4 |
| 18,056 | 4,90904 | 9,2 |
| 18,329 | 4,83643 | 9,2 |
| 18,724 | 4,73540 | 82,7 |
| 19,070 | 4,65016 | 44,1 |
| 19,494 | 4,55001 | 4,5 |
| 20,436 | 4,34235 | 21,4 |
| 20,762 | 4,27483 | 18,4 |
| 21,587 | 4,11339 | 26,7 |
| 22,000 | 4,03705 | 100,0 |
| 22,935 | 3,87457 | 24,2 |
| 23,084 | 3,84979 | 22,3 |
| 23,551 | 3,77450 | 14,0 |
| 23,891 | 3,72152 | 5,6 |
| 24,721 | 3,59850 | 3,6 |
| 25,078 | 3,54803 | 13,2 |

- P027 chloroform solvate, which can be characterized because it has a X-ray powder diffraction pattern showing characteristic peaks at a reflection angle [2θ] of about the values indicated below in table 5:

**Table 5**

| List of selected peaks obtained by powder X-Ray diffraction of the chloroform solvate | | |
|---|---|---|
| **Angle (2θ)** | **value (Å)** | **Intensity (%)** |
| 11,370 | 7,77642 | 10,0 |
| 13,396 | 6,60439 | 0,9 |
| 14,048 | 6,29920 | 1,1 |
| 15,010 | 5,89751 | 33,5 |
| 15,303 | 5,78539 | 4,0 |
| 16,117 | 5,49492 | 4,2 |
| 16,804 | 5,27165 | 1,5 |
| 17,040 | 5,19923 | 6,6 |
| 17,830 | 4,97065 | 2,1 |
| 18,029 | 4,91633 | 8,4 |
| 18,661 | 4,75106 | 11,3 |
| 18,859 | 4,70167 | 7,1 |
| 19,190 | 4,62136 | 2,9 |
| 20,150 | 4,40334 | 3,1 |
| 20,434 | 4,34278 | 1,1 |
| 21,424 | 4,14416 | 2,5 |
| 22,279 | 3,98707 | 100,0 |
| 22,871 | 3,88527 | 16,8 |
| 23,449 | 3,79074 | 9,1 |
| 23,918 | 3,71738 | 0,9 |
| 24,343 | 3,65347 | 3,1 |
| 24,709 | 3,60019 | 4,6 |
| 24,820 | 3,58439 | 1,8 |
| 25,459 | 3,49576 | 16,4 |
| 26,199 | 3,39873 | 7,6 |

The preparation of the above polymorphic and solvated forms represents additional embodiments of the present invention.

P027 phase II form may be obtained in polymer induced crystallizations by solvent evaporation.

Phase III form may be obtained in polymer induced crystallizations either by solvent evaporation or by crystallization by antisolvent addition.

Phase IV form may be obtained in polymer induced crystallizations by crystallization by antisolvent addition.

P027 dioxane solvate may be obtained by solvent drop grinding in dioxane or by crystallization from a hot saturated solution of dioxane.

P027 chloroform solvate may be obtained in polymer induced crystallizations either by solvent (chloroform) evaporation or by crystallization from hot saturated solutions of chloroform.

Another embodiment of the present invention includes the transformation of crystalline forms phase 11, phase III and phase IV into a more stable polymorphic form such as phase I form.

Another embodiment of the present invention includes the transformation of a solvate of P027, preferably chloroform solvate, into a more stable polymorphic form such as phase I form.

A further embodiment of the present invention includes pharmaceutical compositions comprising at least one of the forms of the hydrochloride salt of 4-[2-[[5-methyl-1-(2-naphthalenyl)-1H-pyrazol-3-yl]oxy]ethyl]morpholine above-mentioned i.e. P027 phase 11, phase III, phase IV, chloroform solvate and dioxane solvate.

These aspects and preferred embodiments thereof are additionally also defined in the claims.

### BRIEF DESCRIPTION OF THE FIGURES

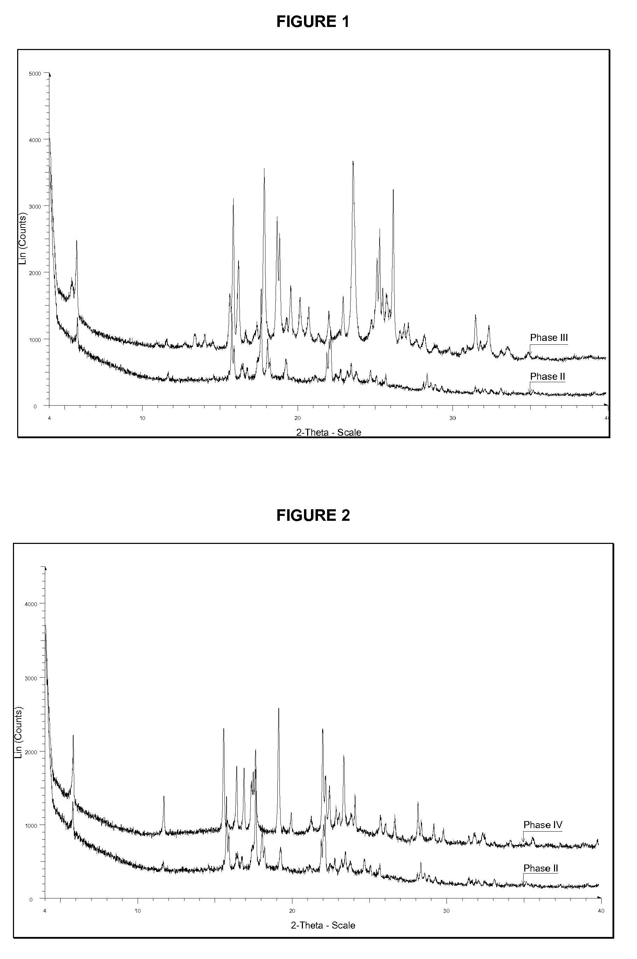

**Fig. 1****:** Comparison of the PXRD patterns obtained for Phase I and Phase III.
**Fig. 2****:** Comparison of the PXRD patterns obtained for Phase II and Phase IV.
**Fig. 3****:** Comparison of the PXRD patterns obtained for Phase III and Phase IV.
**Fig. 4****:** Comparison of the PXRD patterns obtained for Phase I and Phase II.
**Fig. 5****:** Standard PXRD pattern of Phase II.
**Fig. 6****:** ¹H NMR spectrum of Phase II.
**Fig. 7****:** DSC and TGA analyses of Phase II.
**Fig. 8****:** Standard PXRD pattern of Phase 111.
**Fig. 9****:** Comparison of the PXRD patterns obtained for poly(ethylene glycol) and Phase III.
**Fig. 10****:** ¹H NMR spectrum of Phase III.
**Fig. 11****:** ¹H NMR spectrum of poly(ethylene glycol).
**Fig. 12****:** DSC and TGA analyses of Phase III.
**Fig. 13****:** DSC and TGA analyses of poly(ethylene glycol).
**Fig. 14****:** DSC analyses of Phase III with a heating rate of 20 °C/min.
**Fig. 15****:** DSC analyses of Phase III with a heating rate of 30 °C/min.
**Fig. 16****:** Standard PXRD pattern of Phase IV.
**Fig. 17****:** ¹H NMR spectrum of Phase IV.
**Fig. 18****:** DSC and TGA analyses of Phase IV.
**Fig. 19****:** Standard PXRD pattern of the dioxane solvate.
**Fig. 20****:** ¹H NMR spectrum of the dioxane solvate.
**Fig. 21****:** DSC and TGA analyses of the dioxane solvate.
**Fig. 22****:** FTIR analysis of the dioxane solvate.
**Fig. 23****:** Standard PXRD pattern of the chloroform solvate.
**Fig. 24****:** DSC and TGA analyses of the chloroform solvate.

### DETAILED DESCRIPTION OF THE INVENTION

The inventors of the present invention have found novel solid forms of the hydrochloride salt of 4-[2-[[5-methyl-1-(2-naphthalenyl)-1H-pyrazol-3-yl]oxy]ethyl]morpholine (P027) which provides advantageous production, handling, storage and therapeutic properties. The novel P027 compound forms are stable over the time and have good flow and dissolution characteristics and can be formulated and administered providing stable compositions and good pharmacological properties. Additionally, the new forms of P027 may be used for obtaining other forms, such as crystalline phase I form of P027.

As used herein, the term "about" means a slight variation of the value specified, preferably within 10 percent of the value specified. Nevertheless, the term "about" can mean a higher tolerance of variation depending on for instance the experimental technique used. Said variations of a specified value are understood by the skilled person and are within the context of the present invention. Further, to provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including equivalents and approximations due to the experimental and/or measurement conditions for such given value.

As used herein, "room temperature" or its abbreviation "rt" is taken to mean 20 to 25 °C.

The new forms of P027 herein disclosed were characterized by powder X-ray diffraction (PXRD), proton nuclear magnetic resonance (¹H-NMR), differential scanning calorimetry (DSC), thermogravimetric analysis (TGA) and Fourier-transformed infrared spectroscopy. The present invention is directed in one aspect to the new solid forms of P027 in themselves, regardless of the technique used for their characterization. Therefore, the techniques and results provided herein are not intended to limit the present invention, but to serve as characterization of the same. The skilled person will be able, given the guidance and results described herein, to compare and characterize using the available techniques the different polymorphs and solvates of the compound 4-[2-[[5-methyl-1-(2-naphthalenyl)-1H-pyrazol-3-yl]oxy]ethyl]morpholine hydrochloride (P027).

The preparation of solid samples of compound P027 was performed in a set of 40 solvents (table 6). Solvents were selected according to previous experience with the aim to cover a broad range of properties.

**Table 6**

| Solvents used in the crystallization screening with the corresponding codes | | | |
|---|---|---|---|
| **Name** | **Code** | **Name** | **Code** |
| Dimethylsulfoxide | DMS | Diethoxymethane | DEM |
| *N,N*-Dimethylacetamide | DMA | 1,2-Dichloroethane | DCE |
| *N,N*-Dimethylformamide | DMF | Isopropanol | IPH |
| Xylene | XIL | Acetonitrile | ACN |
| Chlorobenzene | CLB | Cyclohexane | CHE |
| *n*-Butanol | NBL | Methyl ethyl ketone | MEC |
| Methyl isobutyl ketone | MIC | Butyl amine | BUA |
| Isobutyl acetate | AIB | Ethanol | EOH |
| Pyridine | PYR | Ethyl acetate | AET |
| Toluene | TOL | 1,1,1-Trichloroethane | TCE |
| 3-Pentanone | POA | *n*-Hexane | HEX |
| Propyl acetate | APR | Diisopropyl ether | DIE |
| Nitromethane | NIM | Tetrahydrofuran | THF |
| Dioxane | DIX | Methanol | MOH |
| Water | H₂O | Chloroform | CLF |
| 2-Butanol | BUL | Methyl acetate | MAC |
| *n*-Heptane | HEP | Acetone | ACE |
| Dimethylcarbonate | CDM | Methyl tert-butyl ether | MTE |
| Triethylamine | TEA | Dimethoxymethane | DMM |
| Isopropyl acetate | AIP | Dichloromethane | DCM |

In order to plan the crystallization screening, the solubility of P027 was determined at room temperature in the set of solvents of table 6 using the following methodology (table 7): 10 mg of the delivered sample were suspended at room temperature in 0.2 mL of the corresponding solvent and successive additions (initially 0.2 mL and finally 0.5 mL) of solvent until the solid was completely dissolved or up to a maximum of 8 mL were performed. After each solvent addition the suspension was vigorously stirred for 10-15 minutes and visually inspected to determine if the solid was completely dissolved. Solubility ranges are listed in table 7.

**Table 7**

| Solubility of P027 in different solvents at room temperature | | | |
|---|---|---|---|
| **Solvent** | **mg/mL** | **Solvent** | **mg/mL** |
| Chloroform | > 50 | Dimethylcarbonate² | 1-2 |
| Dimethylsulfoxide | > 50 | Tetrahydrofuran | < 1.2 |
| Dimethylformamide | > 50 | Methyl acetate | < 1.2 |
| Dichloromethane | > 50 | Isobutyl acetate | < 1.2 |
| Methanol | > 50 | Propyl acetate | < 1.2 |
| Butyl amine | > 50 | Xylene | < 1.2 |
| Water | > 50 | Isopropyl acetate | < 1.2 |
| *N,N*-Dimethylacetamide | 25-50 | Toluene | < 1.2 |
| Nitromethane | 25-50 | Ethyl acetate | < 1.2 |
| Pyridine | 25-50 | 1,1,1-Trichloroethane | < 1.2 |
| Ethanol | 15-25 | Methyl isobutyl ketone | < 1.2 |
| 1,2-Dichloroethane | 15-25 | Methyl tert-butyl ether | < 1.2 |
| Acetonitrile¹ | 10-20 | Dimethoxymethane | < 1.2 |
| *n*-Butanol¹ | 5-10 | Cyclohexane | < 1.2 |
| Acetone | 4.0-5.0 | Chlorobenzene | < 1.2 |
| Isopropanol¹ | 4.0-5.0 | *n*-Heptane | < 1.2 |
| 2-Butanol¹ | 3-4 | *n*-Hexane | < 1.2 |
| Methyl ethyl ketone¹ | 2-4 | Diisopropyl ether | < 1.2 |
| 3-Pentanone² | 1-2 | Triethylamine | < 1.2 |
| Dioxane² | 1-2 | Diethoxymethane | < 1.2 |

| | | | |
|---|---|---|---|
| ¹The solid was dissolved at 60 °C. The solution was left at room temperature and no solid was observed. ²The solid was dissolved at 80 °C. The solution was left at room temperature and no solid was observed. | | | |

The solvents in which P027 was insoluble were used as antisolvents (e.g. those solvents providing a solubility < 1.2 mg/mL). For example, *n*-Heptane (HEP) and diisopropyl ether (DIE) were used as antisolvents. The other solvents were used as dissolving solvents in the different crystallization strategies assayed.

In order to cover the broadest crystallization range possible, several crystallization methodologies were employed using the solvents described in table 6. Procedures oriented to obtain the thermodynamically stable phase as well as procedures directed to obtain kinetically favored phases were used. Moreover, solvent mediated as well as solvent free crystallization procedures were assayed. A list of the crystallization procedures used in this invention is following:
- Solvent evaporation at two rates at room temperature
- Solvent evaporation at different temperatures: -21, 4 and 60 °C
- Crystallization from hot saturated solutions at two cooling rates
- Crystallization aimed to the preparation of hydrates
- Crystallization by addition of an antisolvent
- Crystallization by diffusion of an antisolvent
- Grinding experiments
- Pressure experiments
- Slurry experiments

Additionally to the standard crystallization procedures, a new methodology was used applying polymers to induce the crystallization of new solids. As described in the literature, the use of polymers could favour the formation of new crystalline phases (M. Lang et al. J. Am. Chem. Soc., 2002, 124, 14834.; C. Price et al. J. Am. Chem. Soc., 2005, 127, 5512.). Moreover the presence of polymers could support the formation of larger single crystals and stabilize the formation of solvates. A series of polymers (see table 8) were added in catalytic amounts to a solution of P027 and crystallized using the following methodologies:
- Solvent evaporation at room temperature
- Crystallization from hot saturated solutions
- Crystallization by antisolvent addition
- Grinding experiments

**Table 8**

| Polymers used in this invention | |
|---|---|
| **Name** | **Code** |
| Hydroxipropyl methyl cellulose | HPC |
| Poly(ethylene glycol) | PGY |
| Polyvinyl pyrrolidone | PVP |
| Poly(acrylic acid) | PAA |
| Nylon 6/6 | NYL |
| Polypropylene | PPL |
| Poly(styrene-co-divinylbenzene) | PSV |
| Polyvinylchloride | PVC |
| Poly(tetrafluoroethylene) | PTF |
| Poly(vinyl acetate) | PVA |
| Poly(vinyl alcohol) | PVH |
| Polyacrylamide | PAD |
| Polysulfone | PLS |
| Poly(methyl methacrilate) | PMM |

As used herein referring to polymers, "catalytic amounts" represent a substoichiometric amount of polymer with respect to the compound P027; preferably below a 25% wt of the amount (wt) of compound P027: In a particular embodiment, "catalytic amounts" represent below a 20% wt of the compound P027. In a more particular embodiment, "catalytic amounts" represent below a 10% wt of the compound P027.

All solids obtained using the different crystallization methodologies were characterized by PXRD and classified according to the different PXRD patterns obtained. Further analyses performed were also taken into account for the classification of the solids (see experimental section).

The following forms of P027 were identified and characterized among the solids obtained: P027 phase II form, phase III form, phase IV, dioxane solvate and chloroform solvate.

P027 phase II form, phase III form and phase IV form may be obtained in polymer induced crystallizations either by solvent evaporation or by crystallization by antisolvent addition. Thus, another embodiment of the present invention refers to a process for the preparation of polymorphic forms of the hydrochloride salt of 4-[2-[[5-methyl-1-(2-naphthalenyl)-1H-pyrazol-3-yl]oxy]ethyl]morpholine, comprising:
a) dissolving the hydrochloride salt of 4-[2-[[5-methyl-1-(2-naphthalenyl)-1H-pyrazol-3-yl]oxy]ethyl]morpholine in a suitable solvent or mixture of solvents in the presence of catalytic amounts of a polymer, and
b) either evaporating the solvent or solvents or adding an antisolvent.

In a preferred embodiment, P027 phase II form is prepared by evaporation of a solution of P027 in water with the presence of catalytic amounts of poly(vinyl alcohol).

In another preferred embodiment, P027 phase III form is prepared by evaporation of a solution of P027 in water or acetone with the presence of catalytic amounts of poly(ethylene glycol). P027 phase III form may also be conveniently prepared by addition of diiisopropyl ether as antisolvent to a solution of P027 in water with the presence of catalytic amounts of poly(ethylene glycol).

In another preferred embodiment, P027 phase IV form is prepared by using chloroform as solvent, diisopropyl ether as antisolvent and the following polymers: polyvinyl pyrrolidone (PVP), poly(acrylic acid) (PAA), polypropylene (PPL), poly(styrene-co-divinylbenzene) (PSV), poly(tetrafluoroethylene) (PTF), poly(vinyl alcohol) (PVH), polyacrylamide (PAD) and poly(methyl methacrilate) (PMM).

P027 dioxane solvate may be obtained in a solvent drop grinding experiment in dioxane or by crystallization from a hot saturated solution of dioxane. P027 chloroform solvate may be obtained in polymer induced crystallizations either by solvent (chloroform) evaporation or by crystallization of hot saturated solutions of chloroform.

Thus, another embodiment of the present invention refers to a process for the preparation of solvated forms of the hydrochloride salt of 4-[2-[[5-methyl-1-(2-naphthalenyl)-1H-pyrazol-3-yl]oxy]ethyl]morpholine, comprising at least one of the 3 aternatives i) to iii):
i) a solvent drop grinding, comprising:
   a) charging the hydrochloride salt of 4-[2-[[5-methyl-1-(2-naphthalenyl)-1H-pyrazol-3-yl]oxy]ethyl]morpholine together with catalytic quantities of a suitable solvent to a ball mill container; and
   b) grinding;
ii) crystallization from a hot saturated solution of a suitable solvent; or
iii) a polymer induced crystallization, comprising:
   a) dissolving the hydrochloride salt of 4-[2-[[5-methyl-1-(2-naphthalenyl)-1H-pyrazol-3-yl]oxy]ethyl]morpholine in a suitable solvent in the presence of catalytic amounts of a polymer, and
   b) either evaporating the solvent or crystallizing in a hot saturated solution of the solvent.

In a preferred embodiment, P027 dioxane solvate is prepared by:
a) a solvent drop grinding comprising:
   a) charging the hydrochloride salt of 4-[2-[[5-methyl-1-(2-naphthalenyl)-1H-pyrazol-3-yl]oxy]ethyl]morpholine together with catalytic quantities of dioxane to a ball mill container; and
   b) grinding; or by
b) crystallization from a hot saturated solution of dioxane.

In a preferred embodiment, P027 chloroform solvate is prepared by:
a) dissolving the hydrochloride salt of 4-[2-[[5-methyl-1-(2-naphthalenyl)-1H-pyrazol-3-yl]oxy]ethyl]morpholine in chloroform in the presence of catalytic amounts of a polymer selected from the group consisting of: poly(ethylene glycol), polyvinyl pyrrolidone, poly(acrylic acid), nylon 6/6 , polypropylene, poly(tetrafluoroethylene), poly(vinyl acetate), poly(vinyl alcohol), polyacrylamide and polysulfone; and
b) either evaporating the chloroform or crystallizing in a hot saturated solution of chloroform.

Another embodiment of the present invention includes the use of crystalline forms phase II, phase III and phase IV of P027 in the obtention of the more stable polymorphic phase I form of P027. In one embodiment, the transformation is by heating of crystalline forms phase II, phase III and phase IV into the polymorphic phase I form.

In the DSC analysis of phases II, 111 and IV broad exothermic peaks were observed which correspond to a solid-solid transition. The solid-solid transition (recrystallization) of phase II to phase I was observed at 145 °C. The solid-solid transition (recrystallization) of phase III into phase I was observed in the range 150-170 °C. The solid-solid transition (recrystallization) of phase IV into phase I was observed at 147 °C.

Therefore, in another embodiment the invention is directed to the preparation of phase I form of P027 comprising the step of heating of crystalline forms phase 11, phase III and phase IV of P027 at a temperature between 140°C and 170°C.

Another embodiment of the present invention includes the transformation of a solvate of P027, preferably chloroform solvate, into a more stable polymorphic form such as phase I form. After drying the dioxane solvate for 4 hours at 60 °C, 80 °C and 100 °C the transformation to Phase I was observed. The solids obtained were characterized by PXRD.

A further embodiment of the present invention includes pharmaceutical compositions comprising at least one of the forms of the hydrochloride salt of 4-[2-[[5-methyl-1-(2-naphthalenyl)-1H-pyrazol-3-yl]oxy]ethyl]morpholine above-mentioned i.e. P027 phase II, phase III, phase IV, dioxane solvate and chloroform solvate.

Having described the invention in general terms, it will be more easily understood by reference to the following examples which are presented as an illustration and are not intended to limit the present invention.

### EXAMPLES

Equipment used in the characterization of 4-[2-[[5-methyl-1-(2-naphthalenyl)-1H-pyrazol-3-yl]oxy]ethyl]morpholine hydrochloride solid forms.
a) Powder X-ray Diffraction analysis (PXRD)
   Approximately 20 mg of the non manipulated samples were prepared in standard sample holders using two foils of polyacetate.
   Powder diffraction patterns were acquired on a D8 Advance Series 2Theta/Theta powder diffraction system using Cu_{Kα}-radiation in transmission geometry (Wavelength: 1.54060). The system was equipped with a VANTEC-1 single photon counting PSD, a Germanium monochromator, a ninety positions auto changer sample stage, fixed divergence slits and radial soller. Programs used: Data collection with DIFFRAC plus XRD Commander V.2.5.1 and evaluation with EVA V.12.0.
b) Proton Nuclear Magnetic Resonance (¹H NMR)
   Proton nuclear magnetic resonance analyses were recorded in deuterated chloroform (CDCl₃) in a Bruker Avance 400 Ultrashield NMR spectrometer, equipped with a z-gradient 5 mm BBO (Broadband Observe) probe with ATM and an automatic BACS-120 autosampler. Spectra were acquired solving 2-10 mg of sample in 0.6 mL of deuterated solvent.
c) Differential Scanning Calorimetry analysis (DSC)
   Standard DSC analyses were recorded in a Mettler Toledo DSC822e. Samples of 1-2 mg were weighted into 40 µL aluminium crucibles with a pinhole lid, and were heated, under nitrogen (50 mL/min), from 30 to 300 °C at 10 °C/min. Data collection and evaluation was done with software STARe.
d) Thermogravimetric analysis (TGA)
   Thermogravimetric analyses were recorded in a Mettler Toledo SDTA851 e. Samples of 3-4 mg were weighted (using a microscale MX5, Mettler) into open 40 µL aluminium crucibles with a pinhole lid, and heated at 10 °C/min between 30 and 500 °C, under nitrogen (80 mL/min). Data collection and evaluation was done with software STARe.
e) Fourier Transform Infrared analysis (FTIR)
   The FTIR spectra were recorded using a Bruker Tensor 27, equipped with a MKII golden gate single reflection ATR system, a mid-infrared source as the excitation source and a DTGS detector. The spectra were acquired in 32 scans at a resolution of 4 cm⁻¹. No sample preparation was required to perform the analysis.

### Example 1

### Preparation and characterization of 4-[2-[[5-methyl-1-(2-naphthalenyl)-1H-pyrazol-3-yl]oxy]ethyl]morpholine hydrochloride phase II crystalline form

In the initial screening, a mixture of phase I and a phase II was obtained by solvent evaporation in several solvents (methanol, water, diisopropyl ether-water, nitromethane dioxane-water and heptane-water). This new phase II could be reproduced pure in the screening performed using polymers by evaporation of a solution of P027 in water and with the presence of catalytic amounts of poly(vinyl alcohol).

Crystallization of Form II by solvent evaporation at room temperature: A Sample of 4-[2-[[5-methyl-1-(2-naphthalenyl)-1H-pyrazol-3-yl]oxy]ethyl]morpholine hydrochloride (20-25 mg) was dissolved in the minimum amount of water (0.7 ml) at room temperature and a small quantity of poly(vinyl alcohol) (2-3 mg) was added to the corresponding solution. The resulting solution or suspensions was left to evaporate for two weeks in open vials at room temperature.

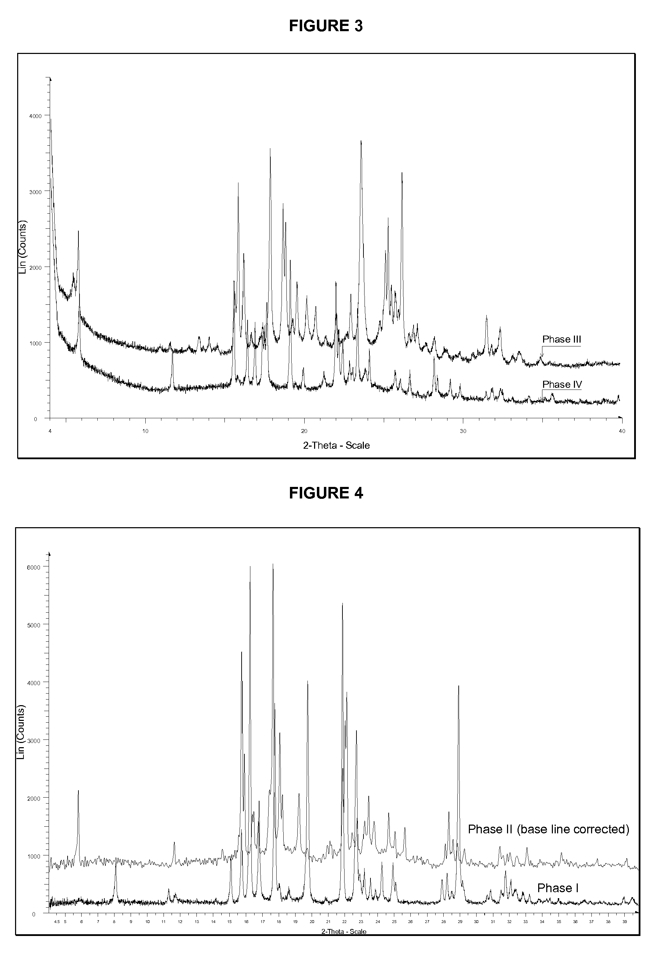

A comparison of the PXRD patterns of phase I and phase II is shown in figure 4. It can be observed that phase II obtained using poly(vinyl alcohol) is pure and no peaks of phase I can be detected in the pattern.

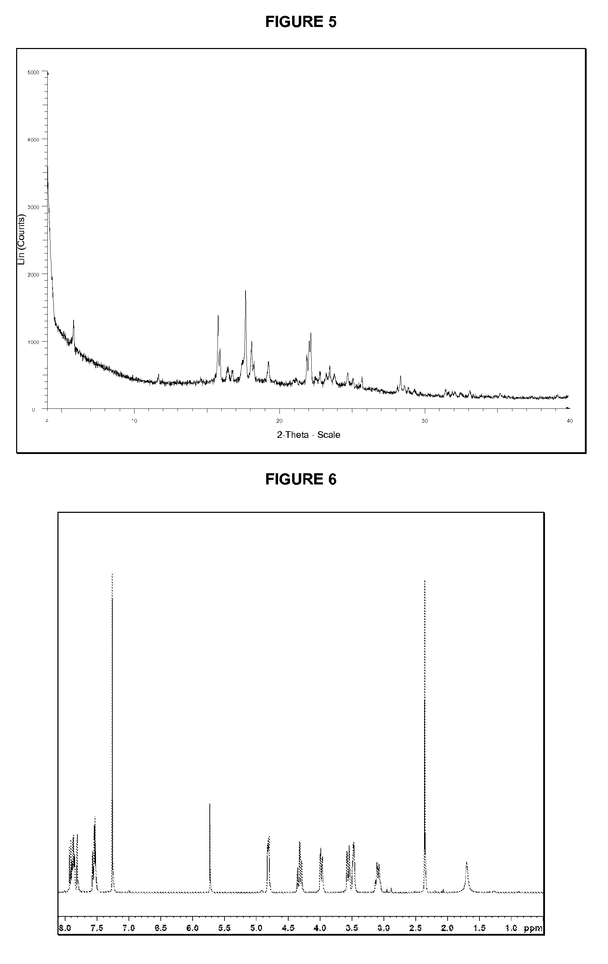

A standard PXRD pattern for phase II is shown in figure 5.

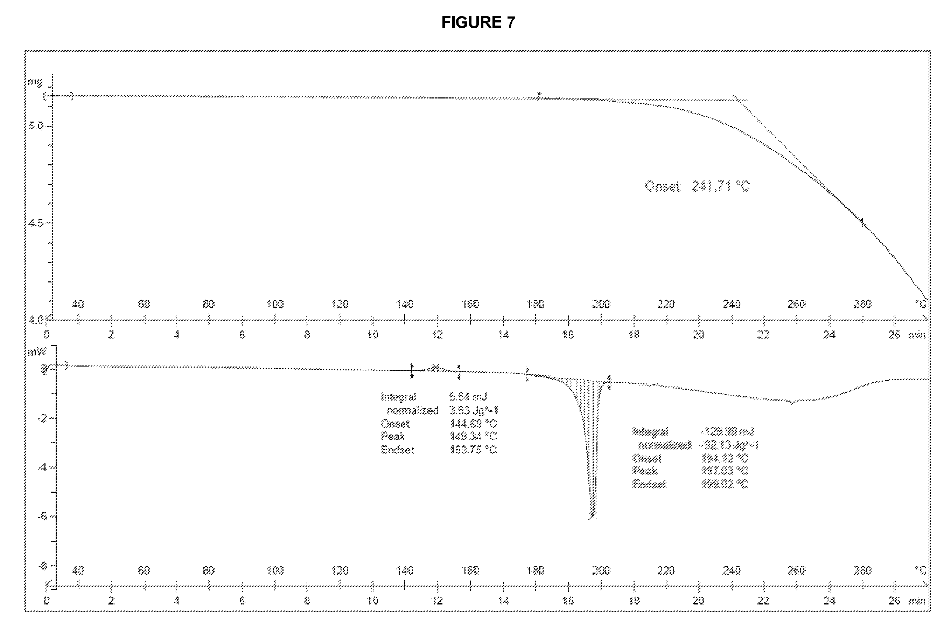

Characterization by ¹H NMR, DSC and TGA is shown in figures 6 and 7.

The ¹H NMR spectrum obtained from the mixture of phases I and II is identical to the one obtained for phase I indicating that phase II is not a decomposition product. The spectra obtained for phase I and phase II are compared in figure 6. No differences in the shifts of relevant hydrogen atoms can be observed.

The DSC analysis of phase II, performed with a heating rate of 10 °C/min, shows a weak broad exothermic peak with an onset at 145 °C and an enthalpy of 4 J/g and a sharp endothermic peak with an onset at 194 °C and an enthalpy of 92 J/g, corresponding to melting followed by decomposition of the product (Figure 7). The small exothermic peak at 145 °C suggests that phase II should be a metastable phase monotropically related to phase I. Thus, the DSC actually shows a solid-solid transition of phase II to I, followed by fusion of phase I.

In the TG analysis of phase II (Figure 7) a weight loss, due to decomposition of the sample, is observed at temperatures higher than 195 °C. The starting temperature of weight loss in the TGA coincides with the melting temperature, confirming that the sample decomposes on melting. No weight loss is observed at temperatures below 180 °C, indicating the absence of solvent. The TG analysis of the solid containing phase II is identical to the one obtained for phase I.

### Example 2

### Preparation and characterization of4-[2-[[5-methyl-1-(2-naphthalenyl)-1H-pyrazol-3-yl]oxy]ethyl]morpholine hydrochloride phase 111 crystalline form

Phase III was generated by polymer induced crystallization. This solid was obtained in four experiments always in the presence of poly(ethylene glycol). In three cases it was obtained by evaporation of water or acetone and in one case it was obtained by addition of diisopropyl ether as antisolvent to a solution in water.

Crystallization of Form III by solvent evaporation at room temperature: A Sample of 4-[2-[[5-methyl-1-(2-naphthalenyl)-1H-pyrazol-3-yl]oxy]ethyl]morpholine hydrochloride (20-25 mg) was dissolved in the minimum amount of water (0.7 ml) or acetone (5.7 ml) at room temperature and a small quantity of poly(ethylene glycol) (2-3 mg) was added to the corresponding solution. The resulting solution or suspensions was left to evaporate for two weeks in open vials at room temperature.

Crystallization of Form III by addition of an antisolvent: A Sample of 4-[2-[[5-methyl-1-(2-naphthalenyl)-1H-pyrazol-3-yl]oxy]ethyl]morpholine hydrochloride (20-25 mg) together with poly(ethylene glycol) (3-4 mg) was dissolved in the minimum amount of water at room temperature and diisopropyl ether (10 ml) was added under vigorous stirring. The final suspension was left evaporate.

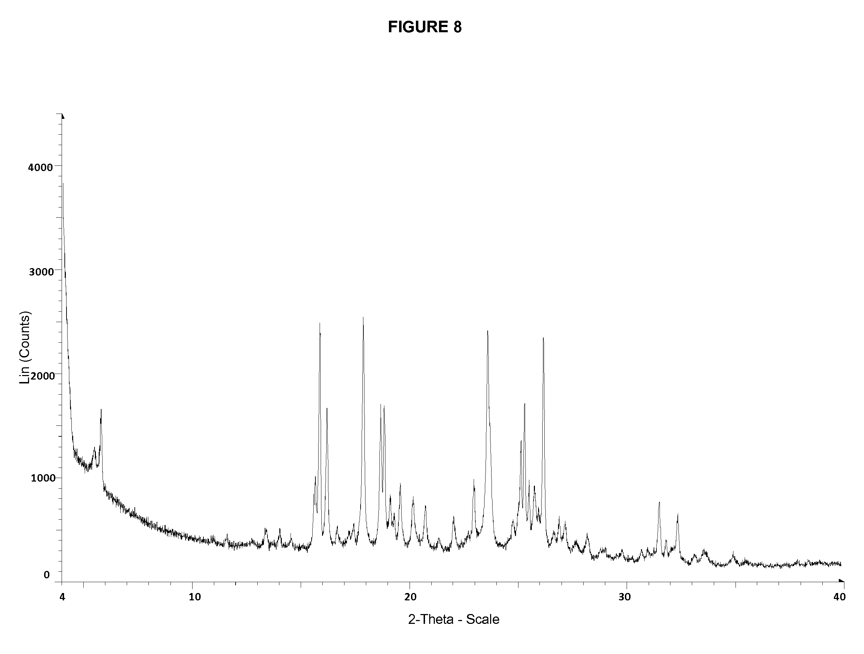

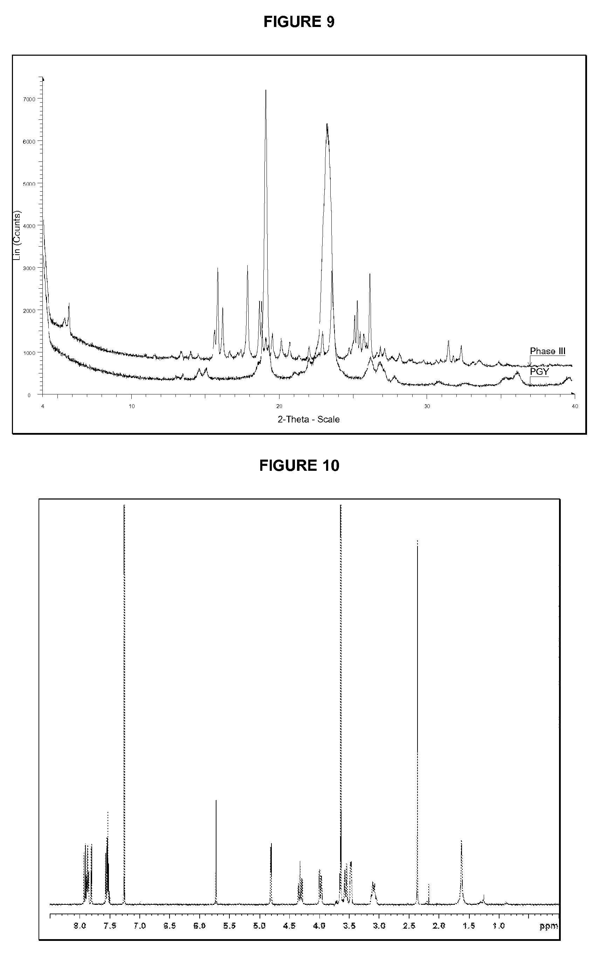

Phase III was characterized by PXRD, ¹H NMR, DSC and TGA. A representative PXRD pattern for phase III is shown in figure 8. By comparing the PXRD pattern of phase III with the pattern of poly(ethylene glycol) the two strongest characteristic signals of the polymer at 19.1° and 23.2° in 2θ can be clearly distinguished (see comparison at figure 9). The peak at 19.1° in 2θ can be observed as a weak signal and the broad peak at 23.2° in 2θ can be also observed slightly shifted to 23.6° in 2θ in the pattern of phase III.

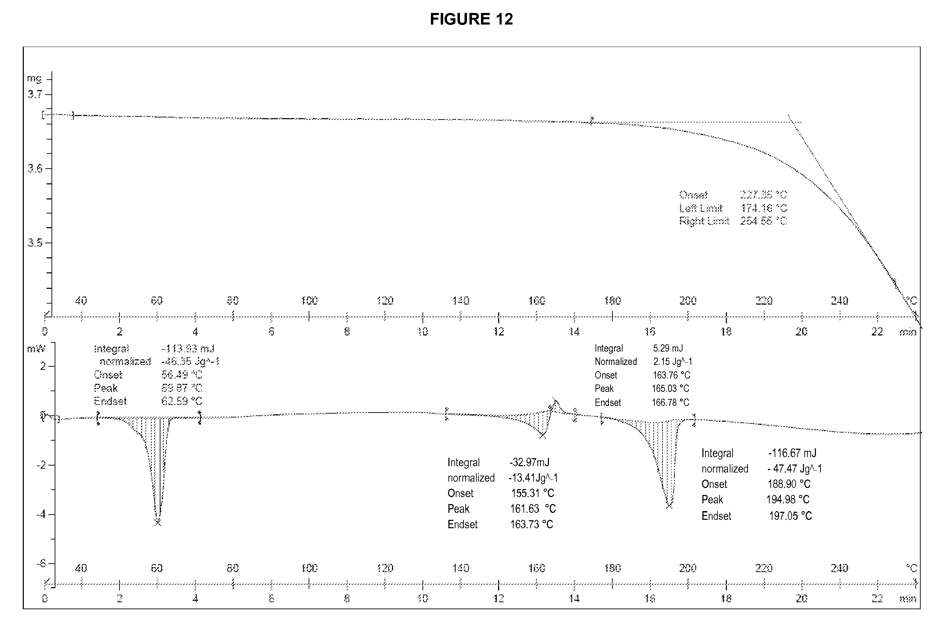

Characterization by ¹H NMR, DSC and TGA is shown in figures 10 and 12.

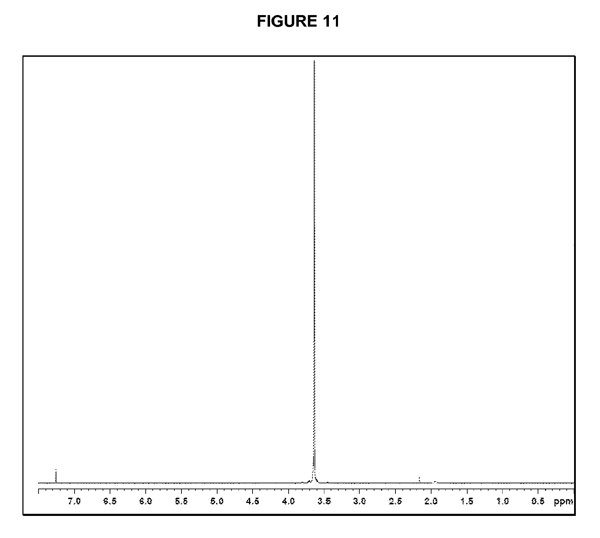

In the ¹H NMR spectrum of phase III the presence of the characteristic signals of P027 indicates that the sample did not decompose. Additionally, in all the spectra measured, the characteristic peak corresponding to poly(ethylene glycol) was observed indicating that phase III is always mixed with this polymer. The ¹H NMR spectrum of poly(ethylene glycol) is represented in figure 11.

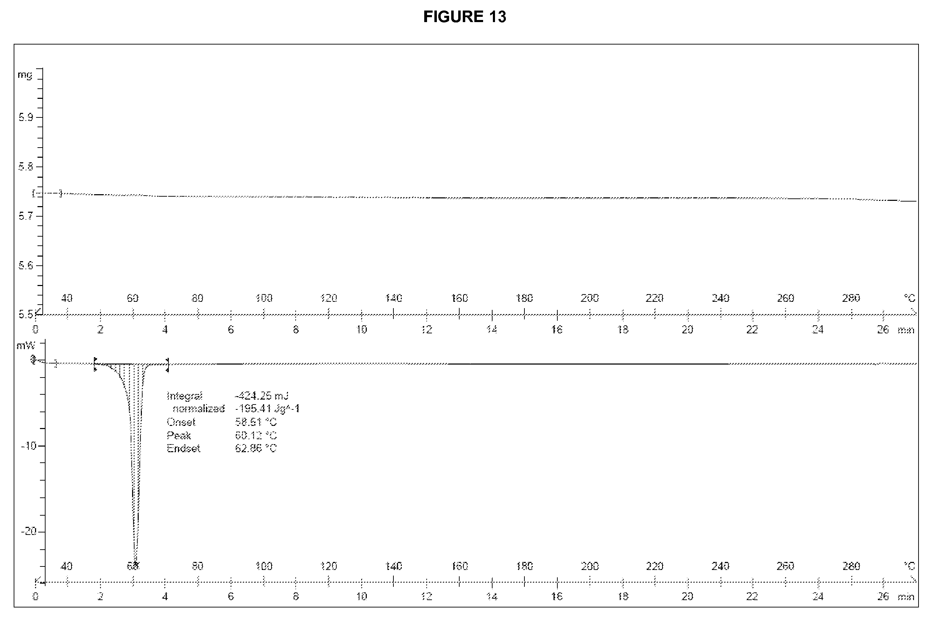

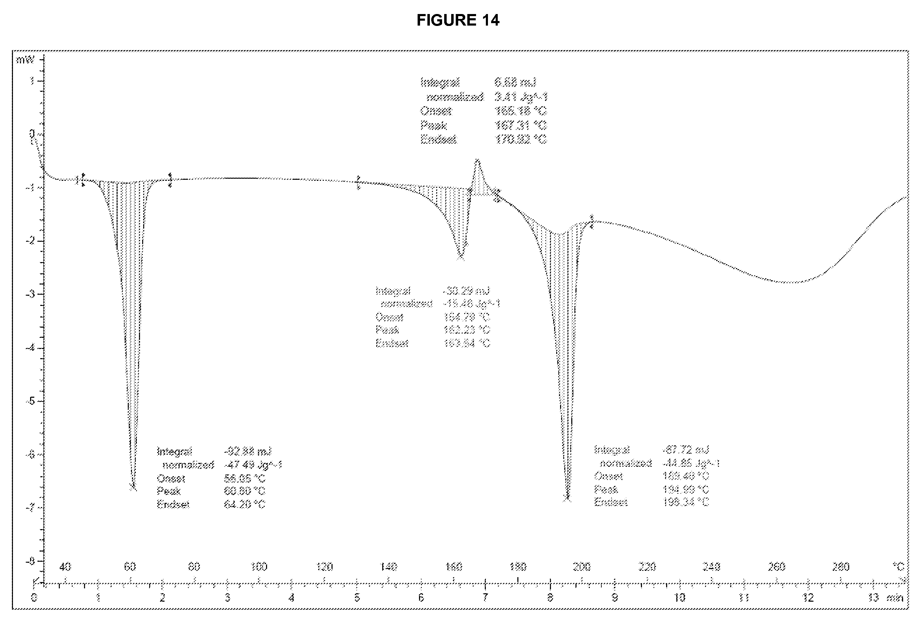

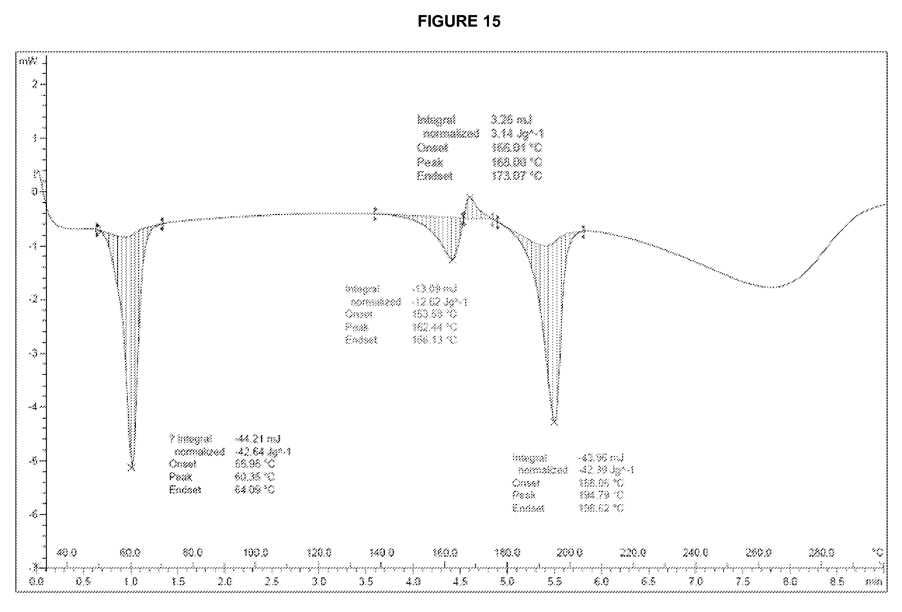

The DSC analysis of Phase III (see figure 12), performed with a heating rate of 10 °C/min, presents a first sharp endothermic peak with an onset at 56 °C and an enthalpy of 46 J/g corresponding to melting of poly(ethylene glycol). The DSC of pure poly(ethylene glycol) is shown in figure 13. In the range from 150 to 170 °C the DSC shows a double peak, first endothermic and then exothermic, corresponding probably to melting of phase III overlapped with recrystallization to phase I. Finally, an endothermic peak with an onset at 190 °C and an enthalpy of 47 J/g, corresponding to melting followed by decomposition of phase I can be observed. Additionally, DSC analyses of the same sample, performed with a heating rate of 20 °C/min (Figure 14) and 30 °C/min (Figure 15) were performed showing that the onset temperature of the endothermic peaks do not vary with the heating rate. This indicates that the endothermic peaks correspond to melting points.

In the TG analysis of Phase III (Figure 12) a weight loss, due to decomposition of the sample, is observed at temperatures higher than 180 °C. No weight loss is observed at temperatures below 180 °C, indicating the absence of solvent. The onset temperature of weight loss in the TGA coincides with the melting temperature, confirming that the sample decomposes on melting.

As used herein, "room temperature" or its abbreviation "rt" is taken to mean 20 to 25 °C.

### Example 3

### Preparation and characterization of 4-[2-[[5-methyl-1-(2-naphthalenyl)-1H-pyrazol-3-yl]oxy]ethyl]morpholine hydrochloride phase IV crystalline form

Phase IV was only generated by polymer induced crystallization. This phase was formed in experiments performed using chloroform as solvent and diisopropyl ether as antisolvent. Phase IV solid was obtained with the following polymers: polyvinyl pyrrolidone (PVP), poly(acrylic acid) (PAA), polypropylene (PPL), poly(styrene-co-divinylbenzene) (PSV), poly(tetrafluoroethylene) (PTF), poly(vinyl alcohol) (PVH), polyacrylamide (PAD) and poly(methyl methacrilate) (PMM). Polymers PVP, PAA, PSV, PVH, PAD and PMM are amorphous and polymers PPL and PTF are crystalline. Only in the sample of phase IV obtained with crystalline PTF, a weak peak of the polymer could be detected in the PXRD pattern.

Crystallization of Form IV by addition of an antisolvent: A Sample of 4-[2-[[5-methyl-1-(2-naphthalenyl)-1H-pyrazol-3-yl]oxy]ethyl]morpholine hydrochloride (20-25 mg) together with 3-4 mg of the corresponding polymer (polyvinyl pyrrolidone, poly(acrylic acid, polypropylene, poly(styrene-co-divinylbenzene, poly(tetrafluoroethylene), poly(vinyl alcohol), polyacrylamide, poly(methyl methacrilate)), was dissolved in the minimum amount of chloroform at room temperature and diisopropyl ether (2 ml) was added under vigorous stirring. The final solid obtained was separated by centrifugation.

Phase IV was characterized by PXRD, ¹H NMR, DSC and TGA.

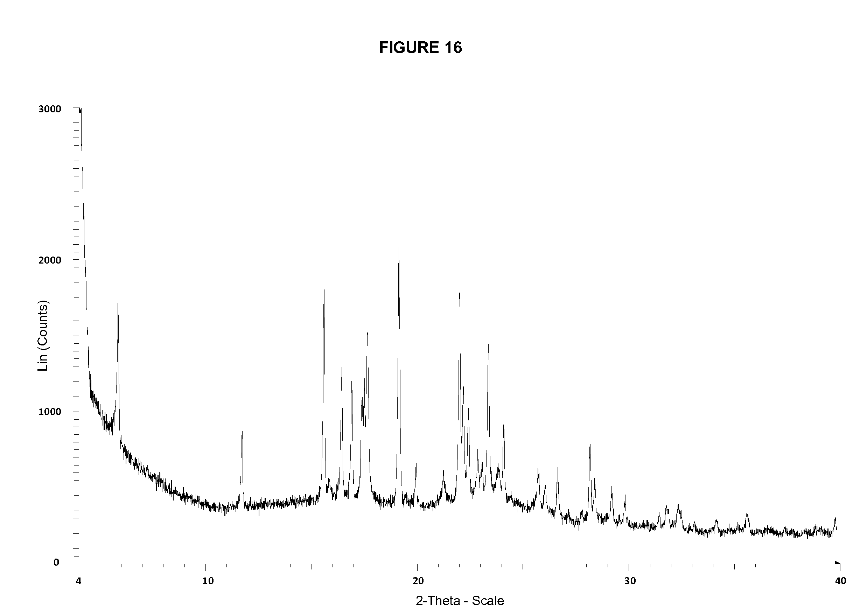

A representative PXRD pattern for Phase IV is shown in figure 16.

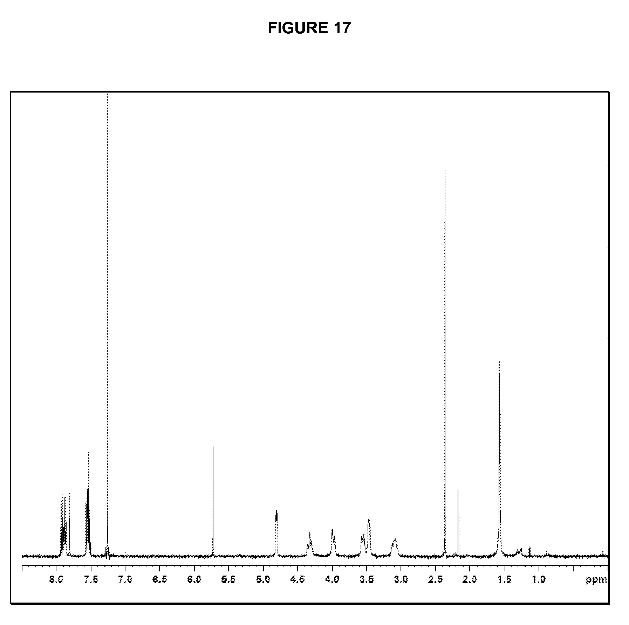

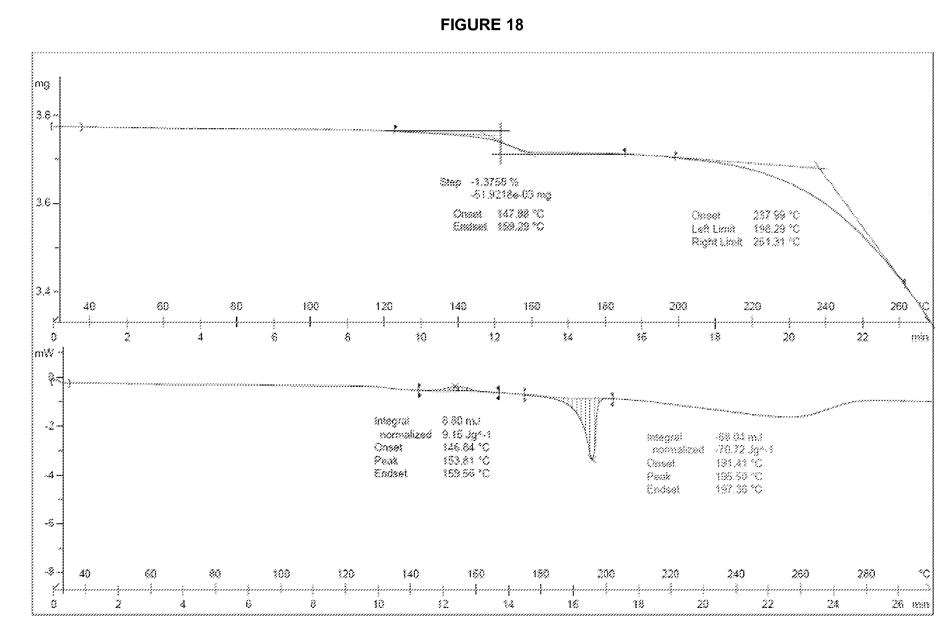

Characterization by ¹H NMR, DSC and TGA is shown in figures 17 and 18.

In the ¹H NMR spectrum of phase IV (see figure 17) the presence of the characteristic signals of P027 indicates that the sample did not decompose. No signals corresponding to the polymers could be detected.

The DSC analysis of Phase IV (see figure 18), performed with a heating rate of 10 °C/min, presents a broad exothermic peak with an onset at 147 °C and an enthalpy of 9 J/g corresponding probably to the solid-solid transition of phase IV to phase I. Finally, an endothermic peak with an onset at 191 °C and an enthalpy of 71 J/g, corresponding to melting followed by decomposition of Phase I can be observed.

In the TG analysis of Phase IV (Figure 18) a small weight loss, corresponding to a 1.4 % of the sample, can be observed between 120 and 170 °C. Decomposition of the sample is observed at temperatures higher than 190 °C. The weight loss probably corresponds to small quantities of water or dichloromethane which is lost in the transition process. The onset temperature of the higher weight loss in the TGA coincides with the melting temperature, confirming that the sample decomposes on melting.

### Example 4

### Preparation and characterization of 4-[2-[[5-methyl-1-(2-naphthalenyl)-1H-pyrazol-3-yl]oxy]ethyl]morpholine hydrochloride solvate with dioxane

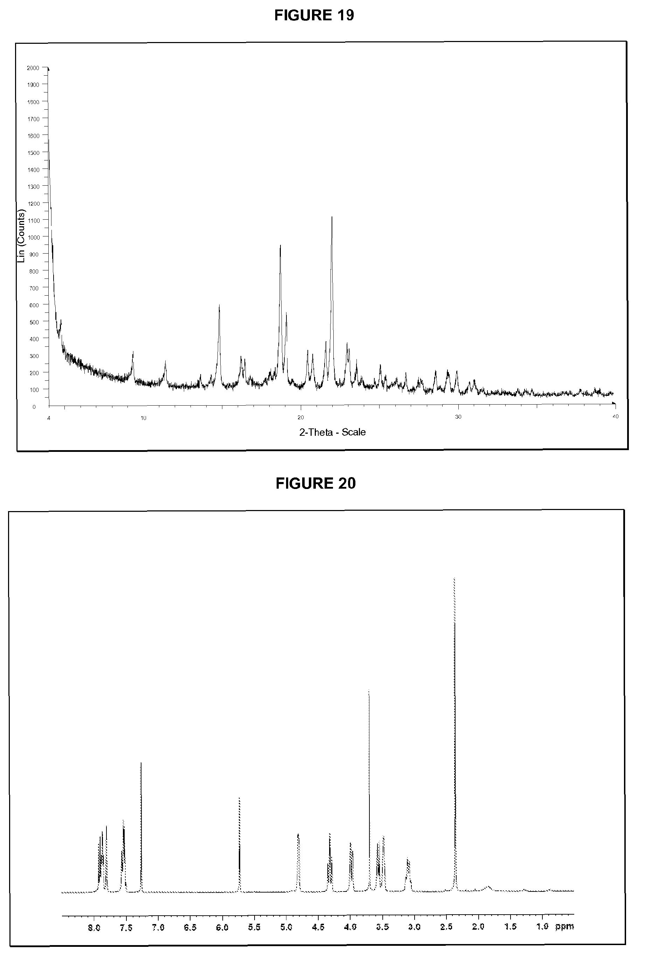

A new crystalline solvated phase, labeled dioxane solvate, was obtained in a solvent drop grinding experiment in dioxane and by crystallization from a hot saturated solution in dioxane. The dioxane solvate crystallizes in form of small sticky crystallites. A representative PXRD pattern of the solvate is shown in figure 19. Characterization by ¹H NMR, DSC, TGA and FTIR is shown in figures 20 to 22.

Grinding experiment: 50 mg of compound together with catalytic quantities of dioxane (three drops) were grinded in a ball mill at 30 s⁻¹ for 30 minutes. For the grinding experiments a Retsch MM400 Ball Mill was used.

Crystallization from a hot saturated solution: 0.5 g of 4-[2-[[5-methyl-1-(2-naphthalenyl)-1H-pyrazol-3-yl]oxy]ethyl]morpholine hydrochloride was dissolved in dioxane (80 mL) at 80 °C. The resulting solution was cooled to 40 °C and a solid started to crystallize. The resulting suspension was kept at 40 °C for 2 hours under gentle stirring, cooled to room temperature and kept at that temperature for 2 hours under gentle stirring. The final solid was filtered off.

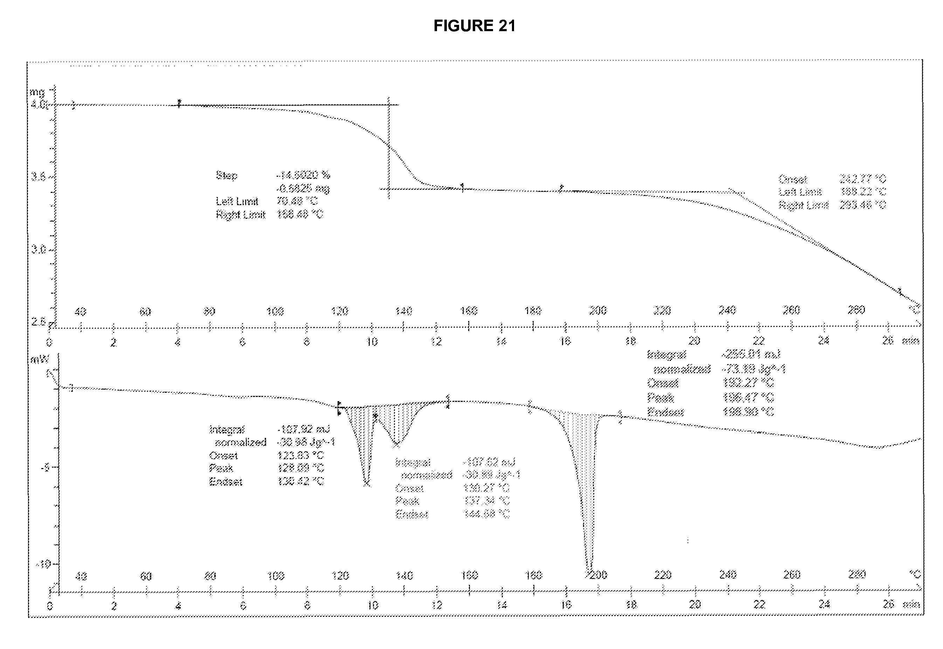

The DSC analysis of the dioxane solvate, with a heating rate of 10 °C/min, presents two overlapped endothermic peaks with onsets at 124 °C and 130 °C, probably due to the loss of dioxane, and a third sharp endothermic peak with an onset at 192 °C and an enthalpy of 73 J/g, corresponding to melting followed by decomposition of the product (Figure 21).

In the TG analysis of the dioxane solvate (Figure 21) a weight loss of 14.6 %, due to the loss of dioxane (theoretical dioxane content for a dioxane monosolvate is 19 %), can be observed between 100 and 160 °C. Decomposition of the sample is observed at temperatures higher than 190 °C. The onset temperature of weight loss due to decomposition in the TGA coincides with the endothermic peak at the DSC, confirming that the sample decomposes on melting. In the ¹H NMR spectrum the characteristic signal of dioxane can be observed confirming the presence of this solvent (see figure 20).

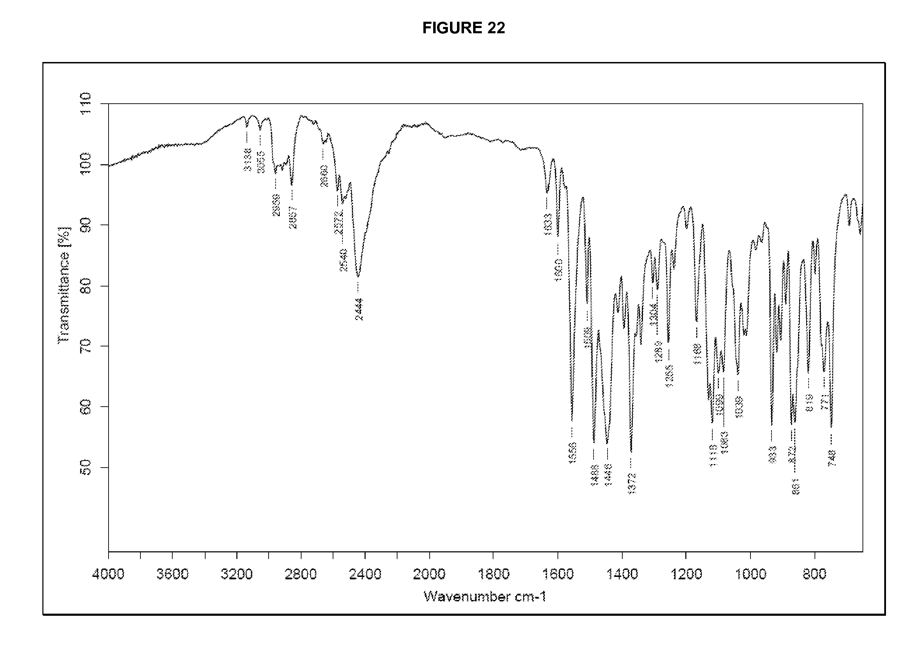

The FTIR spectrum characteristic for the dioxane solvate is represented in figure 22 and presents intense peaks at 3138, 3055, 2959, 2857, 2660, 2572, 2540, 2444, 1633, 1600, 1556, 1509, 1488, 1446, 1372, 1304, 1289, 1255, 1168, 1118, 1099, 1083, 1039, 933, 872, 861, 819, 771 and 748 cm⁻¹.

The scale-up of the dioxane solvate was performed starting from 50, 100 and 500 mg of the compound. The results obtained in each case are gathered in table 9.

**Table 9**

| Scale-up of dioxane solvate | | | | | |
|---|---|---|---|---|---|
| **Entry** | **Scale¹** | **N° exp.** | **Procedure** | **Solvent** | **Observations** |
| 1 | 50 mg | 3 | grinding | dioxane | solvate |
| 2 | 100 mg | 1 | crystallization from hot saturated solution | dioxane | solvate |
| 3 | 500 mg | 3 | crystallization from hot saturated solution | dioxane | solvate |

| | | | | | |
|---|---|---|---|---|---|
| 1)- Referred to starting compound P027 | | | | | |

The solids obtained in the initial screening and in the scale-up at 100 and 500 mg gave the same crystalline phase. At 50 mg scale, the solid was obtained by solvent drop grinding experiments in dioxane. At 100 mg and 500 mg scale, the solid crystallized during cooling to room temperature a hot saturated solution in dioxane.

### Example 5

### Preparation and characterization of 4-[2-[[5-methyl-1-(2-naphthalenyl)-1H-pyrazol-3-yl]oxy]ethyl]morpholine hydrochloride solvate with chloroform.

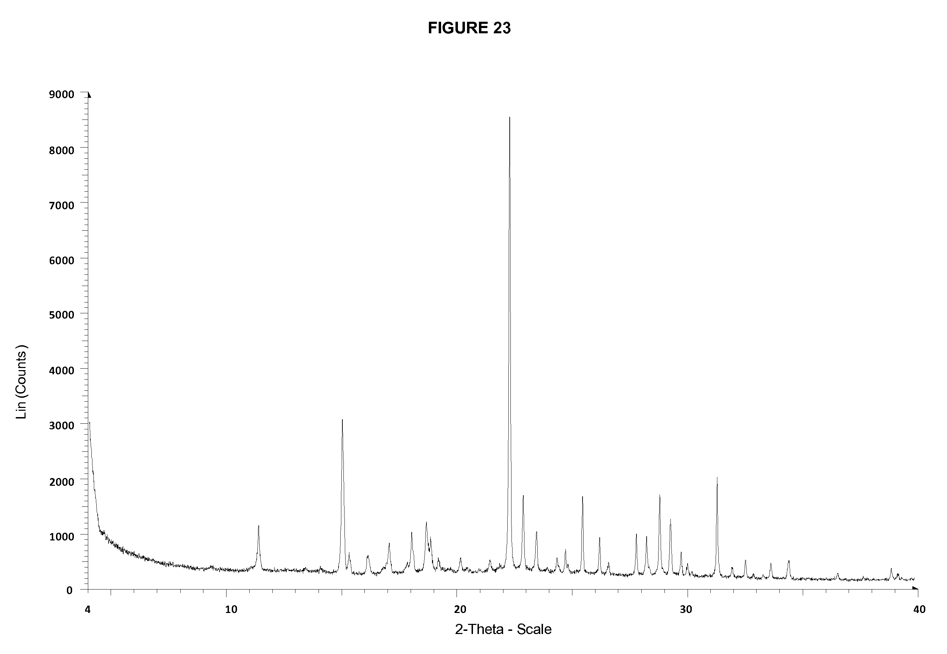

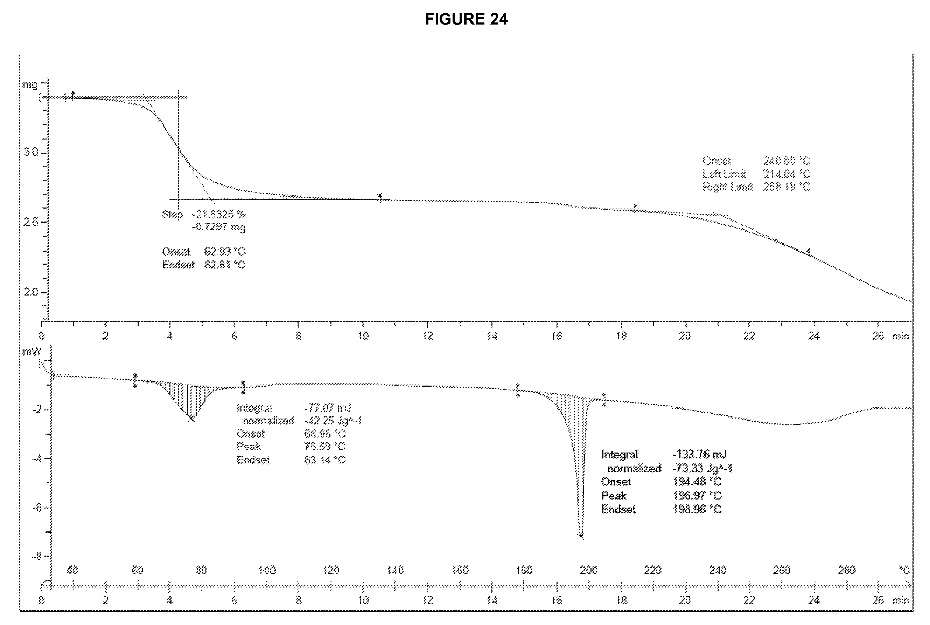

A new crystalline solvated phase, labeled chloroform solvate, was obtained in polymer induced crystallizations. The chloroform solvate of P027 was obtained by evaporation of a chloroform solution or by crystallization of hot saturated chloroform solutions using the following polymers: poly(ethylene glycol) (PGY), polyvinyl pyrrolidone (PVP), poly(acrylic acid) (PAA), nylon 6/6 (NYL), polypropylene (PPL), poly(tetrafluoroethylene) (PTF), poly(vinyl acetate) (PVA), poly(vinyl alcohol) (PVH), polyacrylamide (PAD) and polysulfone (PLS). The polymers PGY, PPL and PTF are crystalline and the rest amorphous. No signals of the crystalline polymers could be observed in the PXRD patterns. The chloroform solvate crystallizes in the majority of the cases in form of large crystals which are probably stabilized by the presence of the polymers. A representative PXRD pattern of the solvate is shown in figure 23. Characterization by DSC and TGA is shown in figure 24.

Crystallization of chloroform solvate by solvent evaporation: A Sample of 4-[2-[[5-methyl-1-(2-naphthalenyl)-1H-pyrazol-3-yl]oxy]ethyl]morpholine hydrochloride (20-25 mg) was dissolved in 0.6 mL of chloroform and 3-4 mg of the corresponding polymer (poly(ethylene glycol), polyvinyl pyrrolidone, poly(acrylic acid), nylon 6/6, polypropylene, poly(tetrafluoroethylene), poly(vinyl acetate), poly(vinyl alcohol), polyacrylamide, polysulfone) was added. The suspension was left to evaporate. After 24 hours, the solid obtained was analyzed by PXRD, DSC and TGA.

The DSC analysis of the chloroform solvate measured with a heating rate of 10 °C/min, presents a broad endothermic peak with an onset at 67 °C and an enthalpy of 42 J/g, due to the loss of chloroform, and a second sharp endothermic peak with an onset at 194 °C and an enthalpy of 73 J/g, corresponding to melting followed by decomposition of phase I (Figure 24).

In the TG analysis of the chloroform solvate (Figure 24) a weight loss of 21.5 %, due to the loss of chloroform (theoretical chloroform content for a chloroform monosolvate is 22.6 %) can be observed between 50 and 120 °C. Decomposition of the sample is observed at temperatures higher than 190 °C.

## Claims

1. A solid form of the hydrochloride salt of 4-[2-[[5-methyl-1-(2-naphthalenyl)-1H-pyrazol-3-yl]oxy]ethyl]morpholine selected from:
- a polymorphic phase II form, preferably having a X-ray powder diffraction pattern showing characteristic peaks at a reflection angle [2θ in degrees] of about 5.776, 11.629, 14.558, 15.737, 15.891, 16.420,16.740, 17.441, 17.635, 18.056, 18.219, 19.232, 19.712, 20.140, 20.685, 21.135, 21.889, 22.108, 22.478, 22.763, 23.219, 23.454, 23.782, 24.689, 25.065, and 25.671;
- a polymorphic phase III form, preferably having a X-ray powder diffraction pattern showing characteristic peaks at a reflection angle [2θ in degrees] of about 5.437, 5.714, 10.918, 11.546, 12.704, 13.344, 13.984, 14.505, 15.606, 15.824, 16.164, 16.646, 17.333, 17.837, 18.719, 18.878, 19.236, 19.533, 20.142, 20.689, 21.337, 22.008, 22.929, 23.596, 24.748, 25.064, 25.207, 25.737, and 26.148;
- a polymorphic phase IV form, preferably having a X-ray powder diffraction pattern showing characteristic peaks at a reflection angle [2θ in degrees] of about 5.805, 11.685, 15.559, 15.804, 16.397, 16.879, 17.357, 17.465, 17.621, 19.112, 19.435, 19.923, 21.224, 21.987, 22.167, 22.412, 2.852, 23.059, 23.359, 23.855, 24.092, 25.722, 26.054, 26.649, and 27.780; and
- a solvated form;
with the 2θ values being obtained using copper radiation (CU_{Kα1} 1.54060 A).

2. The solid form according to claim 1, which is a solvated form selected from:
- dioxane solvate having a X-ray powder diffraction pattern showing characteristic peaks at a reflection angle [2θ in degrees] of about 4.734, 9.317, 11.390, 13.614, 14.290, 14.815, 16.211, 16.432,16.782, 17.741, 18.056, 18.329, 18.724, 19.070, 19.494, 20.436, 20.762, 21.587, 22.000, 22.935, 23.084, 23.551, 23.891, 24.721, and 25.078; and
- chloroform solvate having a X-ray powder diffraction pattern showing characteristic peaks at a reflection angle [2θ in degrees] of about 11.370, 13.396, 14.048, 15.010, 15.303, 16.117, 16.804, 17.040, 17.830, 18.029, 18.661, 18.859, 19.190, 20.150, 20.434, 21.424, 22.279, 22.871, 23.449, 23.918, 24.343, 24.709, 24.820, 25.459, and 26.199;
with the 2θ values being obtained using copper radiation (CU_{Ka1} 1.54060 A).

3. A process for the preparation of the polymorphic phase II form as defined in claim 1, comprising:
a) dissolving the hydrochloride salt of 4-[2-[[5-methyl-1-(2-naphthalenyl)-1H-pyrazol-3-yl]oxy]ethyl]morpholine in water in the presence of catalytic amounts of poly(vinyl alcohol), and
b) evaporating the water.

4. A process for the preparation of the polymorphic phase III form as defined in claim 1, comprising:
a) dissolving the hydrochloride salt of 4-[2-[[5-methyl-1-(2-naphthalenyl)-1H-pyrazol-3-yl]oxy]ethyl]morpholine in water or acetone in the presence of catalytic amounts of poly(ethylene glycol), and
b) evaporating the water or the acetone;
or comprising:
a) dissolving the hydrochloride salt of 4-[2-[[5-methyl-1-(2-naphthalenyl)-1H-pyrazol-3-yl]oxy]ethyl]morpholine in water in the presence of catalytic amounts of poly(ethylene glycol), and
b) adding diisopropyl ether as antisolvent.

5. A process for the preparation of the polymorphic phase IV form as defined in claim 1, comprising:
a) dissolving the hydrochloride salt of 4-[2-[[5-methyl-1-(2-naphthalenyl)-1H-pyrazol-3-yl]oxy]ethyl]morpholine in chloroform in the presence of catalytic amounts of a polymer selected from the group consisting of: polyvinyl pyrrolidone, poly(acrylic acid), polypropylene, poly(styrene-co-divinylbenzene), poly(tetrafluoroethylene), poly(vinyl alcohol), polyacrylamide and poly(methyl methacrilate), and
b) adding diisopropyl ether as antisolvent.

6. A process for the preparation of the dioxane solvate as defined in claim 2, comprising a process selected from:
- solvent drop grinding comprising:
a) charging the hydrochloride salt of 4-[2-[[5-methyl-1-(2-naphthalenyl)-1H-pyrazol-3-yl]oxy]ethyl]morpholine together with catalytic quantities of dioxane to a ball mill container; and
b) grinding;
- crystallization from a hot saturated solution of dioxane.

7. A process for the preparation of the chloroform solvate as defined in claim 2, comprising:
a) dissolving the hydrochloride salt of 4-[2-[[5-methyl-1-(2-naphthalenyl)-1H-pyrazol-3-yl]oxy]ethyl]morpholine in chloroform in the presence of catalytic amounts of a polymer selected from the group consisting of poly(ethylene glycol), polyvinyl pyrrolidone, poly(acrylic acid), nylon 6/6, polypropylene, poly(tetrafluoroethylene), poly(vinyl acetate), poly(vinyl alcohol), polyacrylamide and polysulfone; and
b) either evaporating the chloroform or crystallizing in a hot saturated solution of chloroform.

8. Use of the polymorphic phase II form as defined in claim 1 for obtaining the phase I form.

9. Use of the polymorphic phase III form as defined in claim 1 for obtaining the phase I form.

10. Use of the polymorphic phase IV form as defined in claim 1 for obtaining the phase I form.

11. A process for the preparation of phase I form of the hydrochloride salt of 4-[2-[[5-methyl-1-(2-naphthalenyl)-1H-pyrazol-3-yl]oxy]ethyl]morpholinecomprising the step of heating of crystalline forms phase II, phase III and phase IV of this compound at a temperature between 140°C and 170°C

12. Use of the dioxane solvate as defined in claim 1 for obtaining the phase I form.

13. Use of the chloroform solvate as defined in claim 1 for obtaining the phase I form.

14. A pharmaceutical composition comprising a polymorphic or solvated form as defined in any of claims 1 or 2.
